# EUROPÄISCHE PATENTSCHRIFT

(11) **EP 3 673 838 B1**
(45) Veröffentlichungstag und Bekanntmachung des Hinweises auf die Patenterteilung: **25.11.2020**
(21) Anmeldenummer: 18248060.8
(22) Anmeldetag: 27.12.2018
(51) Int. Cl.: A61B 17/22, A61B 17/00

(54) **DRUCKWELLENGERÄT MIT PROJEKTILANTRIEB**
PRESSURE WAVE DEVICE WITH PROJECTILE DRIVE
APPAREIL À ONDES DE PRESSION POURVU D'ENTRAINEMENT DU PROJECTILE

(43) Veröffentlichungstag der Anmeldung: 01.07.2020
(73) Patentinhaber: STORZ MEDICAL AG, 8274 Tägerwilen (CH)
(72) Erfinder: Heine, Gerold, 78337 Öhningen (DE)
(74) Vertreter: König Szynka Tilmann von Renesse Patentanwälte Partnerschaft mbB

(56) Entgegenhaltungen:
- DE-U1-202014 010 463
- US-A- 5 540 702
- US-A1- 2006 064 864
- US-A1- 2014 350 438

## Beschreibung

Die vorliegende Erfindung betrifft ein Druckwellengerät für die therapeutische Behandlung von Menschen oder Tieren, und zwar ein Druckwellengerät mit einem sogenannten ballistischen Mechanismus. Dabei werden die Druckwellen erzeugt durch die Beschleunigung eines Projektils und dessen Kollision mit einem Applikator (oder sogenannten Übertragungselement) oder auch mit dem behandelten Körper selbst.

Solche Geräte sind seit vielen Jahren vielfach im Einsatz und dienen der Behandlung bei unterschiedlichsten Indikationen. Ursprünglich wurden die Druckwellen durch Kollision mit einem Applikator erzeugt, der sich lang gestreckt und stabförmig aus einem Gerätegehäuse heraus erstreckt hat und zur endoskopischen Einführung z. B. durch die Harnröhre und Behandlung von Konkrementen im Körper, z. B. Nierensteinen, ausgelegt war. Bei anderen Geräten wurden eher flache und großflächigere Frontflächen eines Applikators von außen auf den zu behandelnden Körper aufgelegt.

Dabei werden die Druckwellen in den Körper eingekoppelt, und zwar einerseits in dem Applikator geführte und über die Applikator-Körper-Grenzfläche in diesen eingekoppelte longitudinale "Schallwellen" und andererseits durch eine Schwerpunktbewegung des Applikators, also auch eine Bewegung der Frontfläche des Applikators, in dem Körper selbst erzeugte Wellen. Schließlich sind in Einzelfällen auch Geräte bekannt geworden, bei denen der Applikator gewissermaßen weggelassen wurde und das Projektil unmittelbar auf den zu behandelnden Körper aufschlug. Die vorliegende Erfindung bezieht sich vorzugsweise auf die erste genannte Variante, also mit einem von außen auf den zu behandelnden Körper flächig aufzusetzenden Applikator.

Die Beschleunigung des Projektils ist in der Patentliteratur in unterschiedlicher Weise gelöst, und zwar insbesondere pneumatisch und elektromagnetisch. In der Praxis finden sich im Wesentlichen nur pneumatische Lösungen. Allerdings hat die Firma Zimmer Medizinsysteme zum Zeitpunkt dieser Anmeldung ein Produkt namens "en Puls" mit elektromagnetischem Antrieb im Angebot.

Im praktischen Gebrauch werden seitens der Therapeuten oder Bedienpersonen verschiedene Parameter individuell eingestellt, insbesondere die Intensität der einzelnen Pulse von Druckwellen (d. h. der diese auslösenden Kollisionen) und deren Wiederholfrequenz. Die gewünschten Parameterkombinationen bzw. Leistungsbereiche variieren dabei indikationsabhängig.

Die US 2006/064864 A1 beschreibt ebenfalls ein therapeutisches Druckwellengerät mit einem pneumatischen Antrieb, hier mit besonders hohen pneumatischen Druckwerten. In der US 2014/350438 A1 werden unterschiedliche Mechanismen für die Rückholung des Projektils nach der Kollision angesprochen, unter anderem ein Solenoid-Mechanismus. Zum Stand der Technik wird schließlich verwiesen auf die DE 20 2014 010 463 U1 und auf die US 5,540,702 A.

Aufgabe der vorliegenden Erfindung ist es, ein bezüglich der Einstellbarkeit verbessertes Druckwellengerät anzugeben.

Hierzu richtet sich die Erfindung auf ein Druckwellengerät zur therapeutischen Behandlung des menschlichen oder tierischen Körpers mit einem entlang einer Beschleunigungsstrecke in dem Gerät zu beschleunigenden Projektil zur Erzeugung der Druckwellen durch Kollision des Projektils mit dem zu behandelnden Körper oder mit einem Applikator als Teil des Geräts, welcher Applikator durch die Kollision erzeugte Druckwellen weiterleitet, und mit einem pneumatischen Antrieb zum Beschleunigen des Projektils entlang der Beschleunigungsstrecke, gekennzeichnet durch einen zusätzlichen elektromagnetischen Antrieb zum zusätzlichen Beschleunigen des Projektils entlang der Beschleunigungsstrecke, welcher elektromagnetische Antrieb den pneumatischen Antrieb bei dem Beschleunigen des Projektils unterstützt.

Die Besonderheit der erfindungsgemäßen Lösung liegt in der Kombination eines pneumatischen und eines elektromagnetischen Antriebs. Diese beiden Antriebe sollen dabei nicht nebeneinander im Sinne der Beschleunigung verschiedener Projektile, sondern kombiniert zur Beschleunigung des- oder derselben Projektile eingesetzt werden. Diese auf den ersten Blick redundant und komplex wirkende Lösung zeichnet sich nach den Arbeiten des Erfinders aus verschiedenen Gründen besonders aus.

Bei den im Markt vorherrschenden pneumatischen Antrieben gibt es nach den Erfahrungen des Erfinders gewisse Grenzen hinsichtlich der Kombination hoher Frequenzwerte mit hohen Intensitäten, also relativ großen Werten des pneumatischen Drucks für die Beschleunigung. Das gilt geräteabhängig in unterschiedlichem Ausmaß, aber besonders deutlich für Geräte mit relativ weit von dem zu beschleunigenden Projektil entferntem pneumatischem Schaltventil. Das gilt insbesondere dann, wenn dieses in einem von dem bei der eigentlichen Behandlung bewegten Gerät separaten Basisgerät untergebracht ist. In der Regel bestehen nämlich die in der Praxis verwendeten Geräte aus einem solchen Basisgerät und einem mit einer Leitung daran angeschlossenen Handgerät. Zwar kann in dem Handgerät ein pneumatisches Schaltventil untergebracht werden, und dies ist in der Praxis auch häufig der Fall, jedoch wird das Handgerät einfacher, ökonomischer und in Volumen und Gewicht auch kleiner, wenn das Schaltventil im Basisgerät liegt. Dementsprechend ist dann die Leitungslänge bzw. das Innenvolumen der Leitung mit zu beaufschlagen, wenn der pneumatische Antrieb den beschleunigenden Druck aufbaut.

Wenn nun zusätzlich ein elektromagnetischer Antrieb vorgesehen ist, erlaubt dies eine Beschleunigung des Projektils über das durch den pneumatischen Antrieb (gegebenenfalls ab bestimmten Frequenzen) mögliche Maß hinaus.

Unabhängig von den vorstehenden Überlegungen kann es auch aus anderen Gründen attraktiv sein, die Grenzen des pneumatischen Antriebs zu erweitern, wenn nämlich z. B. nur ein kleiner und ökonomischer (z. B. einstufiger Standard-) Kompressor oder nur eine gängige festinstallierte Pneumatikversorgung mit einem begrenzten Druck zur Verfügung steht, etwa mit einem Druck bis ungefähr 5 bar. Das gilt natürlich auch, wenn aus ökonomischen, Platz- oder Gewichtsgründen nur eine bestimmte Kompressorgröße in Betracht kommt oder vorteilhaft verwendet werden kann.

Umgekehrt stellt sich eine rein elektromagnetische Lösung für den Projektilantrieb nicht als gleichermaßen vorteilhaft wie die Erfindung heraus. Es hat sich nämlich nach den Erfahrungen des Erfinders gezeigt, dass ein typischer elektromagnetischer Antrieb nur begrenzt hohe Projektilgeschwindigkeiten erzielen kann. Aus diesem Grund werden in der Praxis von der bereits erwähnten Anbieterin Zimmer Medizinsysteme relativ schwere Projektile benutzt, um dennoch die gewünschten Energien aufzubringen. Allerdings sind die Druckwelleneigenschaften nicht nur energieabhängig, sondern die Projektilgeschwindigkeit spielt eine wesentliche Rolle. Insoweit gehen die Möglichkeiten eines erfindungsgemäßen Druckwellengeräts über die eines rein elektromagnetischen Antriebs hinaus, jedenfalls bei begrenztem Aufwand.

Die erfindungsgemäße Kombination der beiden Antriebe bedeutet nicht zwingend, dass das Projektil während der gesamten Beschleunigung durch beide Antriebe beaufschlagt wird. Es kann in der Praxis z. B. ausreichend oder vorteilhaft sein, den elektromagnetischen Antrieb nur während einer Teilphase der Beschleunigung zusätzlich einzusetzen, etwa einer späten Phase während der der pneumatische Antrieb wegen des zunehmenden zu füllenden Volumens und der zunehmenden Projektilgeschwindigkeit, mit der sich dieses Volumen vergrößert, weniger wirksam arbeitet. Es ist im Rahmen der Erfindung auch nicht zwingend, wenngleich bevorzugt, während dieser (zweiten) Beschleunigungsphase den pneumatischen Antrieb noch bis zur Kollision oder kurz davor eingeschaltet zu lassen. Eine Kombination der beiden Antriebe bedeutet zunächst einmal nur, dass während ein und derselben Beschleunigung eines Projektils beide Antriebe zum Einsatz kommen.

Insbesondere kann es auch hinsichtlich des Bauvolumens und der Unterbringung anderer Teile des Geräts, beispielsweise einer Gegendruckkammer eines pneumatischen Antriebs (für die Rückführung des Projektils) sinnvoll sein, eine durch den elektromagnetischen Antrieb erfasste Strecke kürzer als die gesamte Beschleunigungsstrecke zu wählen und damit eine Spule dieses Antriebs kürzer auszuführen. Insbesondere kann es sinnvoll sein, die Spule nicht schon am Start der Beschleunigungsstrecke beginnen zu lassen, etwa wenn ein ferromagnetisches Projektil (vergleiche unten) durch die Spule in ihr Inneres hereingezogen werden soll. In diesem Sinn kann man die Spule z. B. eher an das Ende der Gesamtbeschleunigungsstrecke setzen. Das kann auch sinnvoll sein, weil dort die Wirkung des pneumatischen Antriebs wegen der zunehmenden Projektilgeschwindigkeit nachlassen kann und man dann die effektive Beschleunigungsstrecke durch den zusätzlichen elektromagnetischen Antrieb besser nutzen kann. So ist das im Ausführungsbeispiel dargestellt.

Es könnte aber auch bevorzugt sein, die Spule eines elektromagnetischen Antriebs sowohl vom vorderen als auch vom hinteren Ende der Beschleunigungsstrecke etwas zu beabstanden, etwa wenn bei hohen Frequenzen Probleme bestehen, bei einem in einem Basisgerät vorhandenen Schaltventil das Innenvolumen der Leitungen bis zum Projektil schnell genug mit Druck zu füllen und damit die Beschleunigung aufzubauen. Dann kann der elektromagnetische Antrieb auch oder gerade in der Anfangsphase unterstützend wirken.

Insbesondere beträgt der Beschleunigungsstreckenanteil, entlang dem anfangs nur der pneumatische Antrieb wirkt, vorzugsweise mindestens 10 % der gesamten Beschleunigungsstrecke, besonders bevorzugterweise mindestens 20 %.

Eine günstige Implementierung des elektromagnetischen Antriebs sieht ein weichmagnetisches, insbesondere ferromagnetisches Projektil aus oder jedenfalls mit weichmagnetischem, insbesondere ferromagnetischem Material (insbesondere Stahl) in Verbindung mit einer Spule vor. Die Spule wird von der Beschleunigungsstrecke durchsetzt. Wenn die Beschleunigungsstrecke also z. B. (wie üblich und günstig) durch ein gerades langgestrecktes Rohr (Zuführung des pneumatischen Arbeitsgases) gebildet ist, dann umfasst die Spule dieses Rohr zumindest streckenweise. Das magnetische Material hat vorzugsweise eine relative magnetische Permeabilität von mindestens 50, besser 100 oder sogar mindestens 200. Vorzugsweise ist das Rohr selbst nicht ferromagnetisch, also z. B. diamagnetisch oder gar nicht metallisch.

Nach einer weiteren vorteilhaften Ausgestaltung umfasst das Druckwellengerät außerdem einen Sensor zur Erfassung des Projektils in einer bestimmten Position oder generell der Position des Projektils. Vorzugsweise kommen etwa in Betracht ein Abstandssensor, der die Entfernung zwischen dem Sensor und dem Projektil messen kann, z. B. ein Lasersensor, der entlang der Beschleunigungsstrecke ausgerichtet ist, oder ein optischer Sensor in Verbindung mit einer Lichtschranke durch die Beschleunigungsstrecke (also etwa quer dazu) oder auch ein Magnetsensor. Dabei kann es sich z. B. um einen Hall-Sensor in Verbindung mit einem Dauermagneten handeln, wobei das von dem Sensor erfasste Magnetfeld beim Durchgang des Projektils verändert wird. Außerdem kommt auch ein kapazitiver Sensor in Betracht, der die Änderung einer Streukapazität um den Sensor herum durch den Durchgang des Projektils erfasst.

Mit einem Sensor kann also z. B. erfasst werden, dass das Projektil sich der Spule des elektromagnetischen Antriebs nähert oder diese bereits erreicht hat. Dementsprechend kann ansprechend auf ein Sensorsignal eine solche Spule eingeschaltet werden oder auch ausgeschaltet werden. Konkret ist es bevorzugt, bei einer Ausgestaltung mit einer Spule entlang nur einer Teilstrecke der Beschleunigungsstrecke an deren Ende diese Spule nicht von Anfang an gleichzeitig mit dem pneumatischen Antrieb einzuschalten, sondern etwas später, wenn sich das Projektil der Spule schon angenähert hat.

Insbesondere kann die beschleunigende Wirkung z. B. einer Spule auf einer relativ kurzer Strecke (im Ausführungsbeispiel etwa die doppelte Projektillänge) in der Beschleunigungsrichtung beschränkt sein und es insoweit effizienter sein, den elektromagnetischen Antrieb erst dann mit Energie zu versorgen, wenn sich das Projektil schon nennenswert angenähert hat.

Andererseits soll die Spule rechtzeitig ausgeschaltet werden, spätestens bevor das Projektil den von der Spule erfassten Bereich wieder verlässt, damit diese das Projektil nicht bremst. Dementsprechend kann der Sensor auch für das Abschalten statt für das Einschalten benutzt werden oder dazu zusätzlich ein anderer Sensor vorgesehen sein.

Eine weitere Ausgestaltung der Erfindung betrifft eine Mehrzahl Spulen des elektromagnetischen Antriebs entlang der Beschleunigungsstrecke. Diese können dann sequenziell aktiviert werden, um das Projektil dementsprechend effektiver zu beschleunigen. Zur Einstellung der Sequenz der Aktivierung können Sensoren verwendet werden, wie bereits erläutert. Es ist aber auch möglich, bestimmte Zeitverzögerungswerte vorzugeben, eventuell abhängig von der eingestellten Intensität und damit auch der zu erwartenden Projektilgeschwindigkeit.

Das gilt auch allgemein als Ergänzung oder Alternative zu Sensoren: Bei der Konstruktion oder Erprobung eines erfindungsgemäßen Druckwellengeräts können die für bestimmte eingestellte Intensitätswerte zu erwartenden Projektilgeschwindigkeiten umgerechnet werden in (dann im Gerät verankerte) Zeitverzögerungswerte, mit denen ausgehend von bestimmten Zeitpunkten, etwa dem Start des Projektils infolge eines pneumatischen Druckpulsstarts, dann Einschalt- oder Ausschaltvorgänge einer oder einer Mehrzahl Spulen gesteuert werden können. Dies kann in Kombination mit Sensoren geschehen, also beispielsweise ausgehend von einem die Annäherung des Projektils an eine Spule erfassenden Sensor dann mit einer Zeitverzögerung die Spule ausgeschaltet werden, oder auch ganz ohne Sensoren.

Eine erfindungsgemäßes Druckwellengerät kann vor allem bei höheren Betriebsfrequenzen von Vorteil sein und dementsprechend dazu ausgelegt sein, ein Frequenzbereich von beispielsweise 10 Hz bis 25 Hz oder sogar bis 30 Hz zu erreichen, möglicherweise auch noch höhere Frequenzen.

Die bevorzugte Verwendung eines dauermagnetischen oder ferromagnetischen Projektils kann ferner dazu genutzt werden, dieses am Startpunkt der Beschleunigungsstrecke mit einem Dauermagneten in dem Druckwellengerät zu fixieren. Dann muss das Projektil von einem pneumatischen Puls oder elektromagnetischen Puls erst gelöst werden, hat aber zur Anfang des Beschleunigungsvorgangs eine definierte Lage.

Die Rückführung des Projektils erfolgt zusätzlich zu einem kollisionsbedingten Abprallen vorzugsweise pneumatisch. Eine pneumatische Rückführung kann mit einer Gegendruckkammer bewerkstelligt werden, wie sie grundsätzlich aus dem Stand der Technik bekannt ist. Dazu ist das kollisionsseitige Ende der Beschleunigungsstrecke so gestaltet, dass von dem Projektil während der Beschleunigung verdrängtes Pneumatikgas (in der Regel Luft) in die Gegendruckkammer verschoben wird. Z. B. können in einem Führungsrohr für das Projektil an dessen kollisionsseitigem Ende entsprechende Öffnungen vorgesehen sein und kann die Gegendruckkammer in dem Gehäuse des Geräts um dieses Führungsrohr herum angeordnet sein. Es wäre z. B. möglich, die Gegendruckkammer um eine Spule eines elektromagnetischen Antriebs herum vorzusehen oder die Spule in der Gegendruckkammer unterzugbringen.

Andererseits kann auch der elektromagnetische Antrieb für die Rückführung verwendet werden, indem nämlich dessen Spulen sozusagen für eine umgekehrte Bewegung erneut mit einem elektrischen Puls beaufschlagt werden. Wenn die Spule(n) dann rechtzeitig ausgeschaltet wird, kann das Projektil auch über eine eventuell vorhandene spulenfreie Strecke bis zur Ausgangsposition zurückfliegen und dort z. B. von dem erwähnten Magneten gehalten werden. Diese Variante ist ergänzend zu einer pneumatischen Rückführung denkbar, etwa zur Frequenzsteigerung, oder auch alternativ dazu.

Die Erfindung wird im Folgenden anhand eines Ausführungsbeispiels näher erläutert, das in den Figuren dargestellt ist.
- Figur 1: zeigt einen Schnitt durch ein Handstück eines erfindungsgemäßen Geräts;
- Figur 2: zeigt ein Diagramm zur Verdeutlichung der Ergänzung des pneumatischen durch den elektromagnetischen Antrieb;
- Figur 3: zeigt eine Variante der Figur 1 mit einer zusätzlichen Darstellung einer Sensoranordnung zur Projektilerfassung.

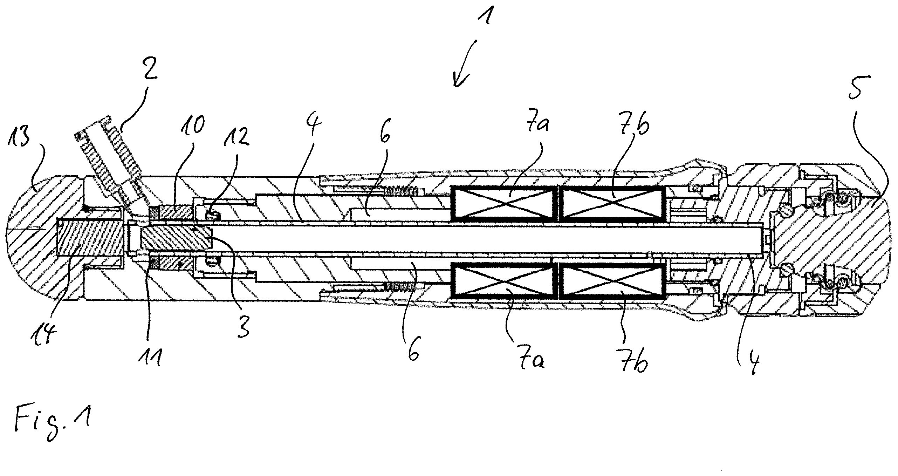

Figur 1 zeigt in einem Längsschnitt entlang der Beschleunigungsrichtung ein Handstück eines erfindungsgemäßen Druckwellenbehandlungsgeräts. Zum Vergleich kann verwiesen werden z. B. auf die US 2011/0054367 A1, die ein ähnliches Handstück, aber nur einen pneumatischen Antrieb dazu (mit einem Schaltventil im Handstück) zeigt.

Auch im vorliegenden Fall wird das insgesamt mit 1 bezifferte Handstück über einen Leitungsanschluss 2 und eine daran angeschlossene flexible Schlauchleitung mit Druckluft aus einem Basisgerät versorgt. Das Basisgerät kann z. B. einen Kompressor beinhalten. Jedenfalls ist bei diesem Ausführungsbeispiel ein nicht gezeigtes Schaltventil zum Ein- und Ausschalten einzelner Druckluftpulse in dem Basisgerät enthalten und beaufschlagt mithin nicht nur das Handstück 1 selbst mit den Druckluftpulsen, sondern auch die gesamte dahin führende Leitung.

Figur 1 zeigt, dass die Druckluftpulse über den Anschluss 2 in ein der Beschleunigung eines Projektils 3 dienendes Rohrstück 4 übertragen werden, wodurch das in Figur 1 rechts am Anfang des Rohrstücks 4 befindliche Projektil 3 entlang dieses Rohrstücks 4 beschleunigt wird. Dadurch kommt es zu einer Kollision mit einem am anderen Ende des Rohrstücks 4 angeordneten Übertragungselement oder einem Applikator 5, der z. B. über ein Kopplungsgel mit einer Patientenkörperoberfläche in Verbindung stehen kann. Dazu ist der Applikator 5 in dem Handstück 1 elastisch aufgehängt, was in der oben zitierten Patentanmeldung näher erläutert ist. Das Rohrstück 4 kann aus nicht magnetischem (d. h. diamagnetischem) Edelstahl bestehen und das Projektil 3 besteht hier aus ferromagnetischem Stahl (umgangssprachlich: magnetischem Stahl), könnte aber grundsätzlich auch ein Permanentmagnet sein oder einen solchen enthalten.

Die während der Bewegung des Projektils 3 auf den Applikator 5 zu vor dem Projektil 3 innerhalb des Rohrstücks 4 verdrängte Luft wird in einer in der Figur nicht genau erkennbaren Weise in eine Gegendruckkammer 6 geleitet, die einen Teil der Erstreckung des Rohrstücks 4 umgibt und, wie im zitierten und anderem Stand der Technik vielfach beschrieben, quasi als pneumatisches Kissen oder als Feder dient. Der darin aufgebaute Druck dient also zur Rückführung des Projektils 3 nach der Kollision in die in Figur 1 dargestellte Position. Soweit ist das Gerät aus dem Stand der Technik bekannt. Im vorliegenden Fall kommen zwei schematisch eingezeichnete elektromagnetische Spulen 7a und b hinzu, die das Rohrstück 4 umgeben und einen Teil von dessen Längserstreckung abdecken, im vorliegenden Fall z. B. 1/3 davon.

Figur 1 zeigt, dass die beiden Spulen 7a, b von beiden Enden des Rohrstücks 4 entfernt angeordnet sind, wobei sie dem applikatorseitigen Ende näher sind. Die Spulen üben in der von sogenannten Ankern bekannten Weise bei Annäherung des Projektils 3 eine anziehende Kraft auf dieses aus, wenn sie mit einem Strom beaufschlagt werden. Diese Kraft zieht das Projektil 3 in das Innere der Spulen 7a, b hinein.

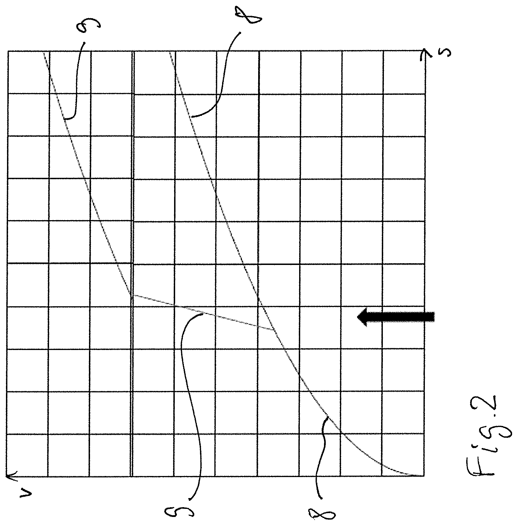

Figur 2 veranschaulicht die zusätzlich zunehmende Projektilgeschwindigkeit durch diese zusätzliche beschleunigende Kraft schematisch (in Bezug auf Figur 1 nicht maßstabsgerecht) in einem Diagramm. Dabei entspricht die Abszisse dem zurückgelegten Weg (Start links im Unterschied zu Figur 1) und die Ordinate der Projektilgeschwindigkeit. Im Sinn einer idealisierten gleichförmigen pneumatischen Beschleunigung bei einem idealisiert konstanten pneumatischen Druck nimmt die Geschwindigkeit als Wurzel aus dem doppelten Produkt aus Beschleunigung und Wegstrecke zu, wobei sich die Beschleunigung aus dem Druck, der Querschnittsfläche und der Projektilmasse errechnet. Auf diesen Zusammenhang 8 kann quasi additiv eine hier als linearer Geschwindigkeitszuwachs idealisierte elektromagnetisehe Beschleunigung aufgesetzt werden, wie die zweite Kurve 9 zeigt, wobei die elektromagnetische Beschleunigungskraft nur über eine recht kurze Strecke bis zum Eintritt in die Spulen 7a, b anhält, was durch den kurzen steilen Abschnitte des Graphen 9 symbolisiert ist. Ab dessen Ende bewegt sich das Projektil dann analog zum Graphen 8, aber mit einem zusätzlichen (elektromagnetisch bewirkten) Geschwindigkeitsbetrag.

Hier ist also pneumatische Beschleunigung parallel zur elektromagnetischen Beschleunigung wirksam, wirkt aber die elektromagnetische Beschleunigung der nur über einen relativ kleinen Teil der Wegstrecke. Diesen Wegstreckenanteil kann man etwas erhöhen, wenn man die beiden Wicklungen 7a und b nicht gemeinsam, sondern separat und zeitlich sequenziell mit einem passenden Strompuls beaufschlagt und natürlich könnte man auch mehr als zwei in dieser Weise separate Spulen in sequenzieller Ansteuerung benutzen.

In jedem Fall ist es erfindungsgemäß möglich, die Projektilgeschwindigkeit über das pneumatisch erzielbare Maß hinaus zu erhöhen. Dementsprechend kann man mit einem begrenzten pneumatischen Versorgungsdruck relativ hohe Projektilgeschwindigkeiten und damit relativ kräftige Stöße erreichen oder bei besonders hohen Frequenzen, wenn insbesondere bei Anordnung des pneumatischen Schaltventils in einem Basisgerät die pneumatische Leitungslänge störend in Erscheinung tritt, für die gewünscht hohen Projektilgeschwindigkeiten Sorge tragen. Zum einen können bei hohen Frequenzen die zeitlichen Verluste beim Druckaufbau durch die Leitung spürbar werden, sodass also das Projektil in der Anfangsphase schwächer beschleunigt wird als gewünscht. Zum anderen kann es bei hohen Projektilgeschwindigkeiten, insbesondere in der Endphase der Beschleunigung, auch zu zunehmenden pneumatischen Verlusten entlang der Leitung kommen.

Daneben wäre es auch möglich, die Einstellung der Projektilgeschwindigkeit rein elektrisch vorzunehmen und die gerätemäßige Einstellung des beschleunigenden Drucks konstant zu halten, was eine Verminderung des technischen Aufwands hinsichtlich der Pneumatik bedeutet. Insoweit könnte man also auf eine Einstellbarkeit des Drucks auch verzichten.

Figur 1 erkennt man, dass die beiden Wicklungen 7a, b in einem Bereich untergebracht sind, in dem bei dem zitierten Stand der Technik Gehäuseteile und die Gegendruckkammer 6 für die Projektilrückführung vorgesehen sind. Im vorliegenden (schematischen) Ausführungsbeispiel nehmen die Wicklungen einen Teil des Platzes der Gegendruckkammer ein. Natürlich könnte der Innenradius der hier zylindrischen Wicklungen auch etwas größer gewählt werden, um mehr Platz für die Gegendruckkammer 6 zu lassen, und könnte das gesamte Gehäuse des Handstücks 1 zumindest in dem Bereich der Wicklungen 7a, b mit Rücksicht darauf radial etwas vergrößert werden; die Wicklungen lassen sich in der Praxis auch radial schmaler ausführen als hier zur Verdeutlichung eingezeichnet.

An der gezeichneten Stelle können sie jedenfalls relativ leicht integriert werden, ohne z. B. die in Figur 1 links davon befindlichen Teile des Handstücks 1 wesentlich verändern zu müssen. Deswegen ist es hier von Vorteil, die Wicklungen 7a, b nicht ganz am linken Ende vorzusehen.

Andererseits kann es auch von Vorteil sein, die Wicklungen 7a, b weiter rechts anzuordnen oder axial entsprechend auszudehnen. Insbesondere könnte damit die Beschleunigung des Projektils 3 in der Startphase der Beschleunigung weiter verbessert werden. Wie eingangs erläutert, kann es bei rein elektromagnetischen Antrieben Probleme geben, hohe Projektilgeschwindigkeiten zu erreichen, weswegen es im Zusammenhang der vorliegenden Erfindung vorteilhaft sein kann, die Wicklungen 7a, b jedenfalls nicht ganz links anzuordnen (bezogen auf Figur 1). Sie sollten einen ausreichenden Abstand vom applikatorfernen Ende der Beschleunigungsstrecke bzw. des Rohrstücks 4 einhalten, um ausreichend Raum für die anziehende elektromagnetische Wirkung auf das Projektil zu lassen.

Im Einzelfall kann dann abhängig von der gewünschten maximalen Stärke der elektromagnetischen Zusatzbeschleunigung und den baulichen Gegebenheiten die nötige axiale und auch radiale Größe der Wicklungen bestimmt werden und können diese dann testweise an verschiedenen axialen Positionen angebracht werden, um ein Optimum zu finden.

Beim vorliegenden Ausführungsbeispiel ist um das in der Ausgangsposition gezeichnete Projektil 3 herum ein Dauermagnet 10 eingezeichnet. Dieser hält das Projektil 3 mit einer relativ schwachen Kraft in der eingezeichneten Position, um eine definierte Lage zum Start eines Beschleunigungs- und Kollisionsvorgangs zu gewährleisten. Der im Stand der Technik umfangreich beschriebene Rückholmechanismus mithilfe des in der Gegendruckkammer 6 durch die Projektilbewegung erzeugten Gegenluftdrucks führt das Projektil 3 in die entsprechende Fixierung durch den Dauermagneten 10 zurück. Im vorliegenden Fall kann das Projektil 3 aus dieser Fixierung mithilfe des pneumatischen Antriebs leicht gelöst werden.

Die ringförmige Struktur 11 rechts von dem Dauermagneten 10 bildet übrigens ein leicht elastisches Gegenlager zum Einklemmen des Dauermagneten 10 an der gezeichneten Stelle, und links von dem Dauermagneten 10 sieht man einen Dichtungsring 12, der letztlich zur Abdichtung der Gegendruckkammer 6 dient.

Noch weiter rechts von dem Projektil 3 findet sich einerseits eine rückseitige Einschraubkappe 13 des Handgeräts 1 und darin mittig und dem Projektil 3 zugewandt ein Lasersensor 14 zur Entfernungsmessung entlang der Beschleunigungsstrecke innerhalb des Rohrstücks 4. Mithilfe dieses Lasersensor 14 kann also die Projektilposition bestimmt werden und damit insbesondere rechtzeitig die Strombeaufschlagung der Wicklungen 7a, b aktiviert bzw. gestoppt werden. Dies ist nötig zur Vermeidung einer Bremswirkung vor der Kollision, weil zwischen den Wicklungen 7a, b und der Kollisionsstelle noch ein deutlicher axialer Abstand besteht.

Außerdem kann mithilfe der gemessenen Position des Projektils 3 die Strombeaufschlagung der Wicklungen 7a, b später eingeschaltet werden als der Start des Beschleunigungsvorgangs bzw. der pneumatischen Druckbeaufschlagung. Im vorliegenden Fall ist die Wirkung der Spule 7 in der Startposition des Projektils 3 und in deren Umgebung ohnehin verschwindend, sodass ein solcher verzögerter Start der elektromagnetischen Beaufschlagung energieeffizienter sein kann. In analoger Weise könnte natürlich bei einer sequenziellen Beaufschlagung einer Mehrzahl von Wicklungen (hier 7a und b) diese sequenzielle Betriebsweise einer Spule mithilfe des Signals des Lasersensors 14 erfolgen.

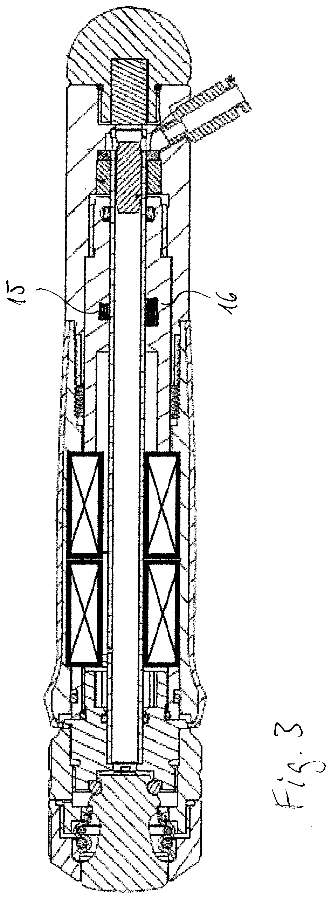

Figur 3 zeigt Alternativen oder zusätzliche Möglichkeiten hierzu, nämlich in Form eines entlang der Beschleunigungsstrecke zwischen der Startposition und der Spule 7 angeordneten Detektors mit zwei Elementen 15 und 16. Z. B. kann es sich bei dem Element 15 um eine kleine Lichtquelle (LED) handeln und kann das Rohrstück 4 an derselben axialen Position zwei einander entlang des Umfangs gegenüberliegende kleine (nicht eingezeichnete) Öffnungen aufweisen (oder transparente Bereiche), sodass das Element 16, nämlich ein Fotodetektor, das Projektil 3 durch die Abschattung des Lichtsignals erfassen kann.

Alternativ könnte es sich bei dem Element 15 um einen kleinen Permanentmagneten handeln, der allerdings so schwach ist, dass die Projektilbewegung nicht wesentlich beeinflusst wird. Dann könnte das Element 16 ein Magnetfelddetektor sein, etwa ein Hallsensor. Der Durchflug des Projektils 3 verändert das Magnetfeld in einer für diesen Sensor 16 erfassbaren Weise. Der Dauermagnet 15 kann eventuell auch weggelassen werden, weil z. B. durch den Magneten 10 bereits ein Magnetfeld vorliegt, dessen Beeinflussung durch das bewegliche Projektil 3 detektierbar ist.

Schließlich könnte es sich bei dem Sensor 16 auch um einen Kapazitätssensor handeln. Die Streukapazität eines solchen Sensors 16 wird durch die Anwesenheit des Projektils 3 verändert, sodass die Streukapazität bei maximaler Annäherung des Projektils 3 einen Extremwert durchläuft. Solche Sensoren sind von der berührungslosen Bedienung von Geräten durch die Detektion der Annäherung von menschlichen Körperteilen bekannt.

Außerdem können, wie bereits weiter oben erläutert, durch die verschiedenen beschriebenen Sensoren erfasste Zeitpunkte oder auch der durch den Start der pneumatischen Beaufschlagung gegebene Zeitpunkt durch Zeitverzögerungsschaltungen umgesetzt werden zur Einschalt- und Ausschaltzeiten für die Wicklungen 7a, b. Die für die Erfassung von Sensorsignalen und gegebenenfalls Zeitverzögerungen sowie im Übrigen auch für die Steuerung des dargestellten Handstücks 1 verantwortliche Steuerung ist vorzugsweise in dem bereits erwähnten Basisgerät untergebracht und über eine elektrische Leitung parallel zu der pneumatischen Leitung mit dem Handstück 1 verbunden.

## Patentansprüche

1. Druckwellengerät zur therapeutischen Behandlung des menschlichen oder tierischen Körpers mit
einem entlang einer Beschleunigungsstrecke in dem Gerät zu beschleunigenden Projektil (3) zur Erzeugung der Druckwellen durch Kollision des Projektils (3) mit dem zu behandelnden Körper oder mit einem Applikator (5) als Teil des Geräts, welcher Applikator (5) durch die Kollision erzeugte Druckwellen weiterleitet,
und mit einem pneumatischen Antrieb (2) zum Beschleunigen des Projektils (3) entlang der Beschleunigungsstrecke,
**gekennzeichnet durch** einen zusätzlichen elektromagnetischen Antrieb (7a, b) zum zusätzlichen Beschleunigen des Projektils (3) entlang der Beschleunigungsstrecke, welcher elektromagnetische Antrieb (7a, b) den pneumatischen Antrieb (2) bei dem Beschleunigen des Projektils (3) unterstützt.

2. Druckwellengerät nach Anspruch 1, bei welchem der elektromagnetische Antrieb (7a, b) dazu ausgelegt ist, das Projektil (3) erst nach Beschleunigung entlang eines Teils der Beschleunigungsstrecke durch den pneumatischen Antrieb (2) dann seinerseits mit einer elektromagnetischen Kraft zu beaufschlagen.

3. Druckwellengerät nach Anspruch 2, bei welchem der Teil der Beschleunigungsstrecke, entlang welchem nur der pneumatische Antrieb (2) wirksam ist, mindestens 10 % der Beschleunigungsstrecke beträgt.

4. Druckwellengerät nach einem der vorstehenden Ansprüche, bei welchem das Projektil (3) zumindest teilweise ferromagnetisch ist und der elektromagnetische Antrieb eine von der Beschleunigungsstrecke durchsetzte Spule (7a, b) aufweist.

5. Druckwellengerät nach einem der vorstehenden Ansprüche, mit einem Sensor (14, 16) zur Erfassung der Position des Projektils (3) zum Einschalten und/oder Ausschalten des elektromagnetischen Antriebs (7a, b) ansprechend auf ein Signal des Sensors (14, 16).

6. Druckwellengerät nach Anspruch 5, bei welchem der Sensor ein, eine Entfernung zwischen dem Sensor (14) und dem Projektil (3) entlang der Beschleunigungsstrecke erfassender Sensor (14), insbesondere ein Lasersensor, ist.

7. Druckwellengerät nach Anspruch 5, bei welchem der Sensor (16) ein optischer Sensor (16) in Verbindung mit einer Lichtschranke (15) ist.

8. Druckwellengerät nach Anspruch 5, bei welchem der Sensor (16) ein magnetischer oder kapazitiver Sensor (16) ist.

9. Druckwellengerät nach einem der vorstehenden Ansprüche, bei welchem der elektromagnetische Antrieb (7a, b) nach Ablauf einer durch eine Zeitverzögerungsschaltung definierten Zeit eingeschaltet und/oder ausgeschaltet wird, und zwar insbesondere nach Auslösung des pneumatischen Antriebs (7a, b) oder nach einem Signal eines Sensors (14, 16) zur Erfassung der Position des Projektils (3).

10. Druckwellengerät nach einem der vorstehenden Ansprüche, mindestens Anspruch 4, mit einer Mehrzahl sequenziell aktivierbarer Spulen (7a, b) entlang der Beschleunigungsstrecke.

11. Druckwellengerät nach einem der vorstehenden Ansprüche mit einem das Projektil (3) und die Beschleunigungsstrecke enthaltenden Handgerät (1) und einem Basisgerät, mit dem das Handgerät (1) über eine pneumatische Leitung verbunden ist, wobei ein pneumatisches Schaltventil des pneumatischen Antriebs in dem Basisgerät angeordnet ist.

12. Druckwellengerät nach einem der vorstehenden Ansprüche ausgelegt für Frequenzen der Beschleunigungsvorgänge des Projektils (3) durch den pneumatischen und durch den elektromagnetischen Antrieb (7a, b) zwischen 10 Hz und 25 Hz.

13. Druckwellengerät nach einem der vorstehenden Ansprüche mit einem Magneten (10), insbesondere einem Permanentmagneten, zur Fixierung des Projektils (3) an einem dem Beginn einer Beschleunigung entsprechenden Ende der Beschleunigungsstrecke.

14. Druckwellengerät nach einem der vorstehenden Ansprüche mit einer Gegendruckkammer (6), wobei das Projektil (3) während der Beschleunigung vor sich befindliches Gas in die Gegendruckkammer (6) drückt und nach der Kollision durch Gas aus der Gegendruckkammer (6) zurückgeführt wird.

15. Druckwellengerät nach einem der vorstehenden Ansprüche, bei dem der elektromagnetische Antrieb (7a, b) dazu ausgelegt ist, für eine Rückführung des Projektils (3) nach einer Kollision zu sorgen.

## Claims

1. A pressure wave device for therapeutic treatment of the human or animal body comprising
a projectile (3) to be accelerated along an acceleration section in the device to generate pressure waves by the collision of the projectile (3) with the body to be treated or with an applicator (5) as part of the device, which applicator (5) forwards the pressure waves generated by the collision,
and comprising a pneumatic drive (2) for accelerating the projectile (3) along the acceleration section,
**characterised by** an additional electromagnetic drive (7a, b) for the additional acceleration of the projectile (3) along the acceleration section, which electromagnetic drive (7a, b) supports the pneumatic drive (2) in accelerating the projectile (3).

2. The pressure wave device according to claim 1, wherein the electromagnetic drive (7a, b) is configured to apply an electromagnetic force to the projectile (3) only after acceleration along a part of the acceleration section by means of the pneumatic drive (2).

3. The pressure wave device according to claim 2, wherein the portion of the acceleration section along which only the pneumatic drive (2) is effective comprises at least 10% of the acceleration section.

4. The pressure wave device according to one of the preceding claims, wherein the projectile (3) is at least partly ferromagnetic, and the electromagnetic drive has a coil (7a, b) through which the acceleration section passes.

5. The pressure wave device according to one of the preceding claims, comprising a sensor (14, 16) for detecting the position of the projectile (3) for switching the electromagnetic drive (7a, b) on and/or off in response to a signal from the sensor (14, 16).

6. The pressure wave device according to claim 5, wherein the sensor is a sensor (14), in particular a laser sensor, detecting a distance between the sensor (14) and the projectile (3) along the acceleration section.

7. The pressure wave device according to claim 5, wherein the sensor (16) is an optical sensor (16) in conjunction with a light barrier (15).

8. The pressure wave device according to claim 5, wherein the sensor (16) is a magnetic or capacitive sensor (16).

9. The pressure wave device according to one of the preceding claims, wherein the electromagnetic drive (7a, b) is switched on and/or off after a time defined by a time delay circuit has lapsed, and in particular after triggering of the pneumatic drive (7a, b) or following a signal from a sensor (14, 16) for detecting the position of the projectile (3).

10. The pressure wave device according to one of the preceding claims, at least according to claim 4, comprising a plurality of sequentially activatable coils (7a, b) along the acceleration section.

11. The pressure wave device according to one of the preceding claims, comprising a handheld device (1) containing the projectile (3) and the acceleration section, and a base device to which the handheld device (1) is connected via a pneumatic line, wherein a pneumatic pilot valve of the pneumatic drive is located in the base device.

12. The pressure wave device according to one of the preceding claims which is configured for the frequencies of between 10 Hz and 25 Hz encountered in the acceleration processes of the projectile (3) initiated by the pneumatic drive and by the electromagnetic drive (7a, b).

13. The pressure wave device according to one of the preceding claims, comprising a magnet (10), in particular a permanent magnet, for fixing the projectile (3) at an end of the acceleration section corresponding to the start of an acceleration.

14. The pressure wave device according to one of the preceding claims, comprising a counterpressure chamber (6), wherein the projectile (3) pushes gas upstream of the projectile into the counterpressure chamber (6) during the acceleration and is returned, after the collision, due to the gas from the counterpressure chamber (6).

15. The pressure wave device according to one of the preceding claims, wherein the electromagnetic drive (7a, b) is configured to return the projectile (3) following a collision.

## Revendications

1. Appareil à ondes de pression destiné au traitement thérapeutique du corps humain ou animal, comprenant:
un projectile (3) destiné à subir une accélération le long d'une section d'accélération au sein de l'appareil afin de produire des ondes de pression sous l'effet de la collision du projectile (3) avec le corps destiné au traitement ou avec un applicateur (5) constitutif de l'appareil, lequel applicateur (5) retransmet les ondes de pression produites par la collision, et
une propulsion pneumatique (2) destinée à faire accélérer le projectile (3) le long de la section d'accélération,
**caractérisé par** une propulsion électromagnétique (7a, b) supplémentaire permettant une accélération supplémentaire du projectile (3) le long de la section d'accélération, laquelle propulsion électromagnétique (7a, b) vient à l'appui de la propulsion pneumatique (2) pour l'accélération du projectile (3).

2. Appareil à ondes de pression selon la revendication 1, dans lequel la propulsion électromagnétique (7a, b) est conçue pour agir sur le projectile (3) avec une force électromagnétique uniquement après son accélération le long d'une partie de la section d'accélération sous l'effet de la propulsion pneumatique (2).

3. Appareil à ondes de pression selon la revendication 2, dans lequel la partie de la section d'accélération le long de laquelle seule la propulsion pneumatique (2) est effective représente au moins 10 % de la section d'accélération.

4. Appareil à ondes de pression selon l'une des revendications précédentes, dans lequel le projectile (3) est au moins partiellement ferromagnétique et la propulsion électromagnétique présente une bobine (7a, b) traversée par ladite section d'accélération.

5. Appareil à ondes de pression selon l'une des revendications précédentes, comprenant un capteur (14, 16) destiné à déterminer la position du projectile (3) afin d'activer et/ou de désactiver la propulsion électromagnétique (7a, b) en réaction à un signal du capteur (14, 16).

6. Appareil à ondes de pression selon la revendication 5, dans lequel le capteur est un capteur (14) déterminant la distance entre ledit capteur (14) et le projectile (3) le long de la section d'accélération, notamment un capteur laser.

7. Appareil à ondes de pression selon la revendication 5, dans lequel le capteur (16) est un capteur optique (16) en liaison avec une barriere lumineuse (15).

8. Appareil à ondes de pression selon la revendication 5, dans lequel le capteur (16) est un capteur (16) magnétique ou capacitif.

9. Appareil à ondes de pression selon l'une des revendications précédentes, dans lequel la propulsion électromagnétique (7a, b) est activée et/ou désactivée au terme d'une durée définie par un circuit de temporisation, notamment après le déclenchement de la propulsion pneumatique (7a, b) ou après un signal d'un capteur (14, 16) permettant de déterminer la position du projectile (3).

10. Appareil à ondes de pression selon l'une des revendications précédentes, au moins selon la revendication 4, comprenant une pluralité de bobines (7a, b) à activation séquentielle le long de la section d'accélération.

11. Appareil à ondes de pression selon l'une des revendications précédentes, comprenant un appareil à main (1) renfermant le projectile (3) et la section d'accélération ainsi qu'un appareil de base auquel l'appareil à main (1) est relié par une ligne pneumatique, une vanne de commande pneumatique de la propulsion pneumatique étant disposée dans l'appareil de base.

12. Appareil à ondes de pression selon l'une des revendications précédentes, conçu pour des fréquences mises en oeuvre lors de l'accélération du projectile (3) sous l'effet des propulsions pneumatique et électromagnétique (7a, b) comprises entre 10 Hz et 25 Hz.

13. Appareil à ondes de pression selon l'une des revendications précédentes, comprenant un aimant (10), notamment un aimant permanent, permettant de fixer le projectile (3) à une extrémité de la section d'accélération correspondant au début d'une accélération.

14. Appareil à ondes de pression selon l'une des revendications précédentes, comprenant une chambre de contre-pression (6), ledit projectile (3) forçant dans la chambre de contre-pression (6), lors de l'accélération, le gaz se trouvant en aval de celui-ci et, après la collision, étant renvoyé par le gaz issu de la chambre de contre-pression (6).

15. Appareil à ondes de pression selon l'une des revendications précédentes, dans lequel la propulsion électromagnétique (7a, b) est conçue pour assurer le renvoi du projectile (3) après la collision.
